(19) Europäisches Patentamt
European Patent Office
Office européen des brevets

(11) EP 1 320 624 B1

(12) **EUROPÄISCHE PATENTSCHRIFT**

(45) Veröffentlichungstag und Bekanntmachung des Hinweises auf die Patenterteilung:
**22.09.2004 Patentblatt 2004/39**

(21) Anmeldenummer: **01969458.7**

(22) Anmeldetag: **20.07.2001**

(51) Int Cl.[7]: **C12P 3/00**

(86) Internationale Anmeldenummer:
**PCT/EP2001/008423**

(87) Internationale Veröffentlichungsnummer:
**WO 2002/010420 (07.02.2002 Gazette 2002/06)**

(54) **SILICATEIN-VERMITTELTE SYNTHESE VON AMORPHEN SILIKATEN UND SILOXANEN UND IHRE VERWENDUNG**

SILICATEIN-MEDIATED SYNTHESIS OF AMORPHOUS SILICATES AND SILOXANES AND USE THEREOF

SYNTHESE ASSISTEE PAR SILICATEINE DE SILICATES ET SILOXANES AMORPHES ET UTILISATION DE CEUX-CI

(84) Benannte Vertragsstaaten:
**AT BE CH CY DE DK ES FI FR GB GR IE IT LI LU MC NL PT SE TR**

(30) Priorität: **28.07.2000 DE 10037270**

(43) Veröffentlichungstag der Anmeldung:
**25.06.2003 Patentblatt 2003/26**

(73) Patentinhaber:
- **MÜLLER, Werner E. G.
65203 Wiesbaden (DE)**
- **Schröder, Heinz C.
65189 Wiesbaden (DE)**
- **Lorenz, Bernd
39110 Magdeburg (DE)**
- **Krasko, Anatoli
55116 Mainz (DE)**

(72) Erfinder:
- **MÜLLER, Werner E. G.
65203 Wiesbaden (DE)**
- **Schröder, Heinz C.
65189 Wiesbaden (DE)**
- **Lorenz, Bernd
39110 Magdeburg (DE)**
- **Krasko, Anatoli
55116 Mainz (DE)**

(74) Vertreter: **Krauss, Jan
Forrester & Boehmert,
Pettenkoferstrasse 20-22
80336 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Silicium ist das zweithäufigste Element der Erdkruste (über 80% der Erdkruste bestehen aus Silikaten) und kommt in verschiedenartigsten Verbindungen vor. Siliciumverbindungen stellen nicht nur die artenreichste Klasse der Mineralien dar, sondern sind auch in wirtschaftlicher Hinsicht außerordentlich wichtig. Sie werden in großem Umfang und in vielfältiger Form eingesetzt. Glas, Porzellan, Email, Tonwaren, Zement und Wasserglas sind technisch wichtige, aus Silikaten bestehende Materialien. Die katalytischen Eigenschaften einiger Silikate werden synthetisch genutzt Ihr vielseitiger Einsatz wird noch erweitert, wenn andere Elemente, insbesondere Aluminium, einige sonst vom Silicium eingenommene Gitterstellen besetzen. Zu diesen Alumosilicaten gehören z.B. die Feldspäte und die Zeolithe; die Bedeutung der letzteren liegt besonders in ihren Molekularsieb- und Ionenaustauscher-Eigenschaften begründet. Al- u. Ca-Silikate haben als Füllstoffe in der Lack-, Kautschuk-, Kunststoff- und Papier-Industrie, Mg-Silikat (Talk) als Absorber- und Füllstoff in der Kosmetik und Pharmazie und Alkali-Aluminium-Silikate als Ersatz für Phosphate in Waschmitteln Bedeutung erlangt. Auch für das Abbinden von Portlandzement spielen Silikate eine wichtige Rolle. Da manche Silikate an ihren Oberflächen freie OH-Gruppen (analog den Silanolen) besitzen, lassen sich dort reaktive Gruppen anbinden; man nutzt diese Eigenschaft in der Festphasen-Technik zur Immobilisierung.

*1.1. Siliciumdioxid*

Siliciumdioxid ($SiO_2$) ist ein Festkörper mit einem hohen Schmelzpunkt, der sowohl in kristallisierter als auch in amorpher Form vorkommt. In allen diesen Erscheinungsformen ist jedes Silicium-Atom tetraedrisch von vier Sauerstoff-Atomen umgeben (Koordinationszahl: 4). Kristallisiert tritt Siliciumdioxid in verschiedenen Modifikationen auf (Quarz, Tridymit, Cristobalit u.a.). Die häufigste Form des kristallinen $SiO_2$ ist der Quarz. Amorphe Siliciumdioxid-Mineralien sind u. a. Achat, Opal und Feuerstein. Quarzglas, das durch Schmelzen von Quarz und langsames Abkühlen der Schmelze erhalten wird, zeigt keine Kristallflächen mehr. Verwendet wird es u.a. zur Herstellung von Quarzlampen (wegen seiner Durchlässigkeit für ultraviolette Strahlen) und hitzebeständiger Apparate. Aus amorphem $SiO_2$ bestehen auch die Schalen von Kieselalgen (Diatomeen).

*1.2. Kieselsäuren und Silicate*

Auch in den Kieselsäuren und Silicaten besitzt Silicium die Koordinationszahl 4. Das tetraedrisch gebaute $[SiO_4]^{4-}$-Ion neigt zur Polymerisation durch Verknüpfung von $SiO_4$-Einheiten. Dabei sind jeweils zwei Si-Atome über ein O-Atom miteinander verbunden.

Durch Wasserabspaltung (Kondensation) entsteht dabei aus der Orthokieselsäure zunächst die Orthodikieselsäure (Pyrokieselsäure; $H_6Si_2O_7$). Die weitere Kondensation führt über die Polykieselsäuren zu den Metakieselsäuren $[(H_2SiO_3)_n]$. Bei kleinerer Zahl der $SiO_4$-Einheiten (n = 3, 4 oder 6) können sich dabei auch ringförmige Moleküle bilden.

Die Polykieselsäuren besitzen einen amorphen (ungeordneten) Aufbau.

Die Salze der Orthodikieselsäuren (Orthosilicate) mit der Zusammensetzung $Me_2SiO_4$ enthalten einzelne $[SiO_4]^{4-}$-Anionen. Die wasserlöslichen Alkalisilicate, welche beispielsweise durch Schmelzen von Quarz mit Soda, Lauge oder Kaliumcarbonat gewonnen werden können, enthalten neben $[SiO_4]^{4-}$- auch $[Si_2O_7]^{6-}$- und $[Si_3O_{10}]^{8-}$-Anionen (und größere Anionen). Solche "Wasserglas"-Lösungen, aus denen die gelösten Teilchen durch Dialyse an einer Membran abgetrennt werden können, dienen u.a. zum Verkitten von Glas und Porzellan, zum Imprägnieren von Papier, als Flammschutzmittel für Holz sowie zur Lebensmittel-Konservierung.

Nach Ansäuerung einer solchen Alkalisilicattösung kondensieren die aus den $[SiO_4]^{4-}$- und $[Si_2O_7]^{6-}$-Gruppen (und größere Gruppen) durch Protonenaufnahme gebildeten Säuremoleküle untereinander zu Polykieselsäuren (siehe oben), wobei die Lösung gelartig wird. Die erhaltenen Polymere bestehen zunächst aus Ketten oder Netzen. Bei weiterem Fortschreiten der Kondensation entstehen dreidimensionale Strukturen, welche der Zusammensetzung $SiO_2$ entsprechen.

Es ergibt sich folgende Einteilung:

1. Silicate mit diskreten Anionen:

   a) Insel-Silicate: Es handelt sich um Ortho-Silicate mit dem Anion $[SiO_4]^{2-}$ Beispiel: Phenakit, Olivin, Zirkon.

   b) Gruppen-Silicate: Die $SiO_4$-Tetraeder sind zu kurzkettigen Einheiten verknüpft. Beispiele: Di-Silicate mit dem Anion $[Si_2O_7]^{6-}$ und Tri-Silicate.

   c) Ring-Silicate: Die $SiO_4$-Tetraeder sind ringförmig angeordnet. Beispiele: Benitoid (3-er-Ring), Axinit (4-er-Ring), Beryll (6-er-Ring).

2. Ketten-Silicate und Band-Silicate. Kettensilicate bestehen aus kettenartig miteinander verbundenen $SiO_4$-Tetraedern; sie sind Polymere des Anions $[SiO_3]^{2-}$. Durch Verknüpfung mehrerer $SiO_4$-Ketten können bandförmige Moleküle entstehen. Beispiele: Hornblenden, Asbeste.

3. Schicht-Silicate (Blatt-Silicate): Schichtsilicaten enthalten ebene Schichten aus $SiO_4$-Tetraedern. Diese werden durch dazwischen gelagerte Kationen zusammengehalten. Sie sind Polymere des Anions $[Si_4O_{10}]^{4-}$. Beispiele: Talk, Kaolinit.

4. Gerüstsilicate: In den Gerüstsilicaten sind die tetraedrischen $SiO_4$-Gruppen zu dreidimensionalen Gittern verbunden. Beispiele: verschiedene Modifikationen von Siliciumdioxid wie Feldspäte.

Der zu den Polykieselsäuren bzw. Polysilikaten führende Kondensationsvorgang läßt sich durch teilweises Ersetzen der OH-Gruppen der Kieselsäure durch einbindige Organylreste, welche sich nicht am Kondensationsvorgang beteiligen, steuern (Herstellung verschiedener Silicone).

Synthetische Kieselsäuren sind amorph, ungiftig und führen im Gegensatz zu den kristallinen $SiO_2$-Modifikationen nicht zur Entstehung einer Silicose.

Allgemeine Literatur:

Hinz, Silicat-Lexikon (2 Bd.), Berlin: Akademie Verl. 1985
Liebau, Structural Chemistry of Silicates, Berlin: Springer 1985
Petzold und Hinz, Einführung in die Grundlagen der Silicatchemie, Stuttgart: Enke 1979
CD Römpp Chemie Lexikon - Version 1.0, Stuttgart/New York: Georg Thieme Verlag 1995

*1.3. Siloxane, Silicone*

Die nach dem Stand der Technik angewandten Methoden zur Darstellung von Silanolen und Siloxanen bestehen darin, daß man auf organische Siliciumderivate, wie zum Beispiel Trimethylsiliciumchlorid $[(CH_3)_3SiCl]$ Wasser einwirken läßt, wobei zunächst Silanole wie zum Beispiel Trimethylsilanol gebildet werden:

$$(CH_3)_3SiCl + H_2O \rightarrow (CH_3)_3Si\text{-}OH + HCl$$

Aus diesen entstehen durch Wasserabspaltung Siloxane wie zum Beispiel Hexamethyldisiloxan $[(CH_3)_3Si\text{-}O\text{-}Si(CH_3)_3]$:

$$2\ (CH_3)_3SiOH \rightarrow (CH_3)_3Si\text{-}O\text{-}SiCH_3)_3 + H_2O$$

Weiterhin lassen sich durch Reaktion von Dimethyl- oder Monomethylsiliciumchlorid mit Wasser hochmolekulare Verbindungen mit ring- oder kettenartigen Strukturen oder dreidimensional vernetzten Makromolekülen ("Silicone") herstellen (über die Zwischenprodukte Dimethylsilandiol bzw. Methylsilantriol):

$$(CH_3)_2SiCl_2 + 2\ H_2O \rightarrow (CH_3)_2Si(OH)_2 + 2\ HCl$$

$$n\ (CH_3)_2Si(OH)_2 \rightarrow (CH_3)_2SiO_n + n\ H_2O$$

Die zur Darstellung weiterer Silicone benutzten Ausgangsverbindungen $R_3SiOH$ (Silanole), $R_2Si(OH)_2$ (Silandiole) und $RSi(OH)_3$ (Silantriole) werden gewöhnlich durch Hydrolyse der entsprechenden Halogenverbindungen $R_3SiCl$, $R_2SiCl_2$ und $RSiCl_3$ hergestellt (R = Ethyl-, Propyl-, Phenyl-Gruppen u.a.).

Siloxane (Silicone) lassen sich demnach einteilen in:

a) Lineare Polysiloxane mit dem Bautyp $R_3SiO[R_2SiO]_nSiR_3$.

b) Verzweigte Polysiloxane, die an den Verzweigungsstellen trifunktionelle oder tetrafunktionelle Siloxan-Einheiten enthalten.

c) Cyclische Polysiloxane, die ringförmig aus difunktionellen Siloxan-Einheiten aufgebaut sind.

d) Vernetzte Polymere, wobei ketten- oder ringförmige Moleküle zu zwei- oder dreidimensionalen Netzwerken verknüpft sind.

Die Viskosität der hochmolekularen, aus kettenförmigen Makromolekülen bestehenden Silicone (Siliconöle) nimmt mit wachsender Kettenlänge zu. Silicone spielen eine bedeutsame Rolle als technische Werkstoffe. In geringem Maß vemetzte Ketten zeigen Kautschuk-Elastizität (Siliconkautschuk; Verwendung: Dichtungen u.a.), stark vernetzte Silicone sind harzartige (Siliconharze).

Aufgrund ihrer, durch den organischen Anteil bedingten hydrophoben (wasserabstoßenden) Eigenschaften finden Silicone zum Imprägnieren (von Textilien, Papier u.a.) Verwendung.

*1.4. Silicatein*

Einige der oben genannten Siliciumverbindungen sind nur kostenintensiv herzustellen bzw. kommen nur in geringen Mengen als Bodenschätze vor und sind deshalb nur unter beträchtlichem Aufwand zu isolieren. Der Prozess der chemischen Synthese der Silikate bedarf dabei drastischer Bedingungen wie hohem Druck und hoher Temperatur.

Organismen (insbesondere Schwämme und Algen) sind im Gegensatz dazu befähigt, mit Hilfe spezifischer Enzyme Silikatgerüste unter natürlichen Bedingungen, d.h. bei niedriger Temperatur und niedrigem Druck, zu bilden. Die Vorteile dieses Syntheseweges sind: hohe Spezifität, koordinierte Bildung, Regulierbarkeit und Möglichkeit der Synthese von Nanostrukturen.

Die Isolierung und Reinigung eines silikatbildenden Enzyms (Silicatein) wurde vor kurzem erstmals beschrieben: Shimizu, K. et al. Proc. Natl. Acad. Sci. USA 95: 6234-6238 (1998).

Hierbei ergibt sich jedoch das Problem, daß die Isolierung und Reinigung des Enzyms (Silicateins) zeit- und arbeitsaufwendig ist und dabei nur relativ geringe Ausbeuten zu erzielen sind.

Ein möglicher Lösungsansatz ist die Synthese des rekombinanten Proteins (rekombinantes Silicatein) mit Hilfe der bekannten cDNA- oder Gen-Sequenz. Dies erlaubt die effektive enzymatische Synthese von Silikaten.

Bei der Herstellung des rekombinanten Silicateins aus den Meeresschwämmen *Suberites domuncula* und *Tethya aurantia* ergab sich jedoch das Problem, daß bei Anwendung der dem Stand der Technik (Shimizu, K. et al. Proc. Natl. Acad. Sci. USA 95: 6234-6238 (1998), Shimizu, K. et al., Patentanmeldung, Veröffentlichungsnummer WO0035993 (1999)) entsprechenden Methoden nur sehr geringe Ausbeuten erzielt werden konnten und das rekombinante Protein nur geringe enzymatische Aktivität zeigte. Die vorliegende Erfindung beschreibt, daß durch gezielte Veränderung der Expressionsbedingungen rekombinantes Silicatein in hohen Ausbeuten und mit hoher spezifischer Aktivität hergestellt werden kann. Darüber hinaus weist das modifizierte rekombinante Enzym eine höhere pHund Temperaturstabilität auf als das natürliche und das rekombinante mit kompletter c-DNA Sequenz. Das modifizierte rekombinante Protein besitzt zudem eine enzymatische Aktiviatät in einem weitem pH - Bereich auf (4.5-10) im Gegensatz zu dem natürlichen und rekombinantem Protein mit kompletter c-DNA Sequenz, das bei pH-Werten im neutralen Bereich (pH 7,0) aktiv ist.

Durch die Herstellung eines spezifischen polyklonalen Antikörpers und anschließende Koppelung an eine feste Phase kann eine schnelle und effektive affmitätschromatographische Reinigung des Enzyms durchgeführt werden.

Durch Einsatz von Fusionsproteinen und Verwendung verschiedener Ausgangssubstrate ergeben sich zahlreiche Variationsmöglichkeiten und technische Anwendungen.

## 2. Herstellung von Silicatein

### *2.1. Herstellung von rekombinantem Silicatein*

2.1.1. Clonierung der cDNAs aus marinen Schwämmen

Das Silicatein-Protein von Schwämmen enthält charakteristische Sequenz-Segmente, von denen einige genannt seien: Bereich um die Serin-reichen Aminosäure-Cluster (bei S. *domuncula*: Aminosäuren 267-277); Bereich um das Cystein, der ersten Aminosäure der katalytischen Triade (bei *S. domuncula:* Aminosäure 138), die bei Silicatein fehlt und bei den verwandten Enzymen, den Cathepsinen, vorhanden ist; Bereich um den Übergang zwischen dem Propeptid und dem prozessierten Peptid (bei *S. domuncula:* Aminosäuren 112/113).

Aus cDNA-Bibliotheken, z. B. in *ZapExpress* und in *Escherichia coli* XL1-Blue MRF', kann dann mit geeigneten degenerierten Primern (beispielsweise: der reverse Primer 5'-GAA/GCAG7CCGIGAIGAA/GTCA/GTAG/CAC-3' zusammen mit dem 5'-Vektor-spezifischen Primer [Bereich um die Aminosäuren 267-277] - oder der Vorwärtsprimer am gleichen Protein-Segment zusammen mit dem 3'-Vektor-spezifischen Primer) das Gen für Silicatein mittels der Technik der Polymerasen-Ketten Reaktion identifiziert werden; hierzu wird ein entsprechender vektorspezifische Primer eingesetzt. Das erhaltene Syntheseprodukt wird zum Screenen in der betreffenden cDNA-Bibliothek benutzt. Danach wird der/die identifizierte(n) Clon(e) in einen Vektor (beispielsweise *pGem-T*) subcloniert und anschließend sequenziert. Abbildung 1 zeigt als Beispiel die cDNA für Silicatein aus *Suberites domuncula*.

2.1.2. Expression und Isolierung des rekombinantem Silicatein

Die Herstellung von rekombinantem Silicatein erfolgt bevorzugt in *E. coli* XL1-Blue. Aber auch die Herstellung in Hefen und Säugerzellen ist möglich und wurde erfolgreich durchgeführt. Hierzu wird die cDNA in einen entsprechenden Vektor, z. B. *pQE-30*, eincloniert. Auch andere Expressionsvektoren haben sich als geeignet erwiesen. Nach Transformation von *E. coli* wird die Expression von Silicatein durch Induktion mit üblicherweise IPTG (Isopropyl-β-D-thiogalactopyranosid) (Ausubel, F.M., Brent, R., Kingston, R.E., Moore, D.D., Smith, J.A., Seidmann, J.G. & Struhl, K. (1995) *Current Protocols in Molecular Biology.* John Wiley and Sons, New York) durchgeführt. Die Expression von Silicatein sowie die Aufreinigung des rekombinanten Proteins über z. B. das Histidin-Tag, das an dem rekombinanten Protein vorliegt, kann an entsprechenden Affinitätssäulen, z. B. einer Ni-NTA-Matrix durchgeführt werden (Skorokhod, A., Schäcke, H., Diehl-Seifert, B., Steffen, R, Hofmeister A., and Müller W.E.G. Cell. Mol. Biol. 43:509-519; 1997).

Die Expression der kompletten Silicatein-Sequenz in *E. coli* gelingt jedoch nur mit sehr geringen Ausbeuten. Folgende Veränderungen der Expressionsbedingungen führten unerwarteterweise zu drastischen Verbesserungen der Ausbeute:

1. Durch Verkürzung der Sequenz, die für das N-terminale Ende des Proteins codiert, wurde eine über 100-fache Erhöhung der Expression (im Vergleich zur Expression des Gesamt-cDNA) erreicht Bevorzugt wird der Start in die Region des Propeptides gesetzt. Aber auch eine Verkürzung im Bereich des "reifen" Protein vor der ersten potentiellen Disulfid-Brücke (üblicherweise an der Aminosäure 135) führt zu der gewünschten starken Expression (Abbildung 2).
2. Auch durch eine verkürzte cDNA, die für das C-terminale Ende des Proteins codiert, wird im Vergleich zur Expression der kompletten cDNA eine über 20-fache erhöhte Expression von Silicatein gefunden (Abbildung 2). Erfolgreich hat sich die Verkürzung des Genes am 3'-Ende im Bereich der Sequenz nach der letzten potentiellen Disulfid-Brücke (üblicherweise an der Aminosäure 319) erwiesen. Dort wird ein Stop-Codon insertiert.

Nach Anwendung dieser einzusetzenden Verfahrensänderungen wird überraschenderweise eine hohe Expression in *E. coli* erreicht (mehr als 100-fach); jetzt werden Ausbeuten von etwa 10 mg/100 ml Bakterien-Kulturmedium an rekombinantem Silicatein erreicht (Abbildung 3).

2.1.3. Expression und Isolierung des rekombinantem Silicatein aus anderen silikatbildenden Organismen

Entsprechend der oben beschriebenen Vorgehensweise kann die Isolierung, Clonierung und Expression der cDNA für Silicatein aus weiteren Siliciumdioxid-produzierenden Organismen durchgeführt werden, beispielsweise aus Diatomeen (z. B. *Cylindrotheca fusiformis*). Die Gewinnung von Diatomeen in axenischen Kulturen ist Stand der Technik (Kröger, N., Bergsdorf, C., and Sumper M. Europ. J. Biochem. 239:259-264; 1996).

*2.2. Isolierung und Reinigung von Silicatein aus Tieren und Einzellern*

Silicatein ist dasjenige Enzym, das amorphes Silikat, z. B. in Schwämmen, synthetisiert. Deshalb kann das Silicatein auch aus den Organismen selbst gewonnen werden. Hierzu werden, z. B. aus dem Schwamm *Suberites domuncula* die Spiculae (bestehend aus amorphem Silikat) durch Dissoziation des Gewebes in $Ca^{++}$- und $Mg^{++}$-freiem Seewasser gewonnen. Die Spiculae werden durch Sedimentation gewonnen. Das amorphe Silikat der Spiculae wird im alkalischen Milieu, z. B. in verdünnter Natronlauge, entfernt. Die organischen Fibrillen der Spiculae, die das Silicatein enthalten, werden durch Abzentrifugation (z. B. 20,000 x g; 1 Stunde; 4°C) gewonnen. Das Protein wird durch hohe Salzkonzentration, wie z. B. 1 M NaCl, aber auch durch den "Protein Refolding-Kit" in Lösung gebracht.

Anschließend wird das Silicatein an einer Affinitätsmatrix gereinigt. Die Affintitätsmatrix wird hergestellt, indem ein Silicatein-spezifischer Antikörper an eine feste Phase (CNBraktivierte Sepharose oder andere geeignete Träger) immobilisiert wird, gereinigt. Als Antikörper werden monoklonale oder polyklonale Antikörper gegen das Silicatein eingesetzt, die nach Standard-Methoden hergestellt werden (Osterman, L.A. Methods of Protein and Nucleic Acid Research Vol. 2; Springer-Verlag [Berlin] 1984). Die Kopplung des Antikörpers an die Säulenmatrix wird nach den Angaben des Herstellers (Pharmacia) durchgeführt. Die Elution des reinen Silicateins erfolgt mittels pH-Änderung oder Änderung der Ionenstärke. Auch andere Affinitätsmatrizes wie polymere Silikate oder copolymere Silikate/Germanate wurden mit Erfolg eingesetzt. Die Elution des Silicateins von diesen Matrizes geschieht bei einem pH um den isoelektischen Punkt von Silicatein (im Falle des *S. domuncula*-Silicateins bei einem pH von etwa 6).

Entsprechende Isolierungen von Silicatein aus weiteren Siliciumdioxid-produzierenden Organismen, wie Diatomeen (z. B. *Cylindrotheca fusiformis*), wurden nach der oben dargestellten Verfahren mit Erfolg durchgeführt.

Neu an der Erfindung ist außerdem, daß das Silicatein-Gen durch geeignete Silikatkonzentrationen im Medium (gewöhnlicherweise 60 μM) und durch Myotrophin (1 μg/ml) induziert werden kann (Abbildung 4A,B).

3. Nachweis der Silicatein-Aktivität und Synthese von Silicium-Alkoxy-Verbindungen

Die Messung der enzymatischen Aktivität des rekombinanten Silicateins wird üblicherweise wie folgt durchgeführt. Das rekombinante Silicatein wird über Nacht gegen einen für die Reaktion geeigneten Puffer, wie 25 mM Tris-HCl, pH 6,8 dialysiert [andere Puffer innerhalb eines pH-Bereiches von 4,5 bis 10,5 sind ebenfalls geeignet].

Üblicherweise werden 1 - 60 μg rekombinantes Silicatein in 1 ml eines geeigneten Puffers, wie 25 mM Tris-HCl (pH 6,8) gelöst und mit 1 ml einer üblicherweise 1 - 4,5 mM Tetraethoxysilan-Lösung versetzt. Die enzymatische Reaktion kann bei Raumtemperatur - aber auch bei Temperaturen zwischen 5°C und etwa 65°C - durchgeführt werden. Die durchschnittliche Inkubationszeit ist 60 min. Während dieser Zeitspanne werden üblicherweise 300 nmol amorphes Silikat (als Molybdat-reaktives, lösliches Silikat) pro 100 μg Silicatein synthetisiert. Zum Nachweis der Silikat-Produkte wird das Material in einer Tischzentrifuge abzentrifugiert (12 000 x g; 15 min; +4°C), mit Ethanol gewaschen und luftgetrocknet. Anschließend wird das Sediment mit z. B. 1 M NaOH hydrolysiert. In der entstandenen Lösung wird unter Anwendung eines Molybdat-gestützten Nachweisverfahrens, wie z. B. dem Silicon-Assays (Merck), Silikat quantitativ gemessen.

Überraschenderweise wurde gefunden, daß Silicatein neben dem Substrat Tetraethoxysilan auch noch weitere Silan-Alkoxide polymerisiert.

Neu in dem erfindungsgemäßen Verfahren ist, daß folgende Verbindungen zur Silicatein-vermittelten Synthese verwendet werden können: Tetraalkoxysilane, Trialkoxysilanole, Dialkoxysilandiole, Monoalkoxysilantriole, Alkyl- oder Aryl-Trialkoxysilane, Alkyl- oder Aryl-Dialkoxysilanole oder Alkyl- oder Aryl-Monoalkoxysilandiole oder die entsprechenden (Alkyl- oder Aryl-substituierten) Alkoxyverbindungen von Gallium (IV), Zinn (IV) oder Blei (IV). Auch Mischungen dieser Substrate werden durch das Enzym erkannt und polymerisiert. Somit können auch Mischpolymere hergestellt werden.

Zur Erhöhung der Aktivität des Enzyms Silicatein werden die Substrate, wie Tetraethoxysilan, in Dimethylsulfoxid in einer Stammlösung von üblicherweise 500 mM gelöst und anschließend in die gewünschte Endkonzentration herunter verdünnt.

4. Kopplung der cDNA für Silicatein mit einer oder mehreren cDNA(s) (offene Leserahmen) für anderer Proteine

*4.1. Herstellung von Silicatein-Fusionsproteinen*

Fusionsproteine mit Silicatein werden wie folgt gewonnen. Ein geeigneter Expressionsvektor (beispielsweise *pQE-30*) wird eingesetzt Die Silicatein-cDNA - mit z. B. einer *Bam*HI-Restriktionsstelle am 5'-Terminus und z. B. einer *Sal*I-Restriktionsstelle am 3'-Terminus - wird hergestellt. Das Stop-Codon in der Silicatein-cDNA wird entfernt. Hierzu wird die PCR-Technik eingesetzt und Primer, welche die betreffenden Restrikionsstellen besitzen, zum Amplifizieren benutzt. Die cDNA für das zweite Protein wird entsprechend gewonnen, wobei am 5'-Terminus die gleiche Schnittstelle wie am 3'-Terminus der Silicatein-cDNA (im Beispiel *Sal*I) und am 3'-Terminus eine von den anderen verschiedene (z. B. eine *Hin*dIII-Stelle) vorliegt. Falls sich in den betreffenden cDNAs interne Restriktionsstellen befinden, können alternative Restriktionsenzyme eingesetzt werden. Darüber hinaus können auch Linker zwischen die beiden cDNAs eingesetzt werden (Abbildung 5A).

Diese beiden cDNAs werden nach den üblichen Verfahren ligiert, gereinigt und in den pQE-30 Vektor (Quiagen) einligiert. Die Ligation erfolgt im Anschluß an das Histidin-Tag (etwa 6 Histidin-Codons). Die Expression und Reinigung des Fusionsproteins kann wie oben beschrieben erfolgen (Abschnitt 2.1.2).

*4.2. Getrennte Expression I*

Alternativ zu dem Verfahren, das unter 4.1. beschrieben wurde, kann zwischen der cDNA für das Silicatein und der cDNA für das bioaktive Protein eine Protease-Spaltstelle (wie z. B. eine Enterokinase-Stelle) eincloniert werden. In diesem Falle kann ein Codon für ein neues Start-Methionin vor den codierenden Bereich des Genes für das bioaktive Protein insertiert werden. Nach Expression und Reinigung wird das (Fusions)-Protein proteolytisch gespalten.

Jetzt liegen beide Proteine separat vor (Abbildung 5B).

*4.3. Getrennte Expression II (Kassetten-Expression)*

Alternative können beide Proteine auf einem Konstrukt - aber separat - exprimiert werden. Hierzu wird in einem Expressions-Vektor das Silicateingen dem His-Tag nachgeschaltet. Am Ende der Silicatein-cDNA wir ein Stop-Codon insertiert. Zwischen der cDNA für das Silicatein und der cDNA für ein bioaktives Protein wird eine Ribosomen-Bindungsstelle mit Codon für ein Start-Methionin eincloniert. Wiederum wird ein His-Tag der cDNA für das bioaktive Protein vorgeschaltet. Ebenfalls erhält dieses Gen ein Stop-Codon (Abbildung 5C).
Die His-Tags können deletiert werden, wenn die Proteine zur Funktionsanalyse in den betreffenden Wirtszellen benutzt werden.

*4.4. Erweiterungen*

Für die Expression, wie unter 4.1 bis 4.3 beschrieben, können sowohl bakterielle als auch eukaryotische Zellen benutzt werden.
Die Expression, wie unter 4.1 bis 4.3 beschrieben, kann auch für drei und mehr offene Leserahmen eingesetzt werden.

5. Verwendungen der hergestellten Silicateine und Silicatein-Fusionsproteine

*5.1. Verwendung von Silicatein zur Oberflächenmodifikation von Biomaterialien*

Die biologische Reaktion von Organismen (oder biologischen Extrakten/Produkten) auf (exponierte und/oder implantierte) Biomaterialien wird großenteils durch deren Oberflächenstruktur und -chemie bestimmt. Deshalb besteht ein Bedarf an Methoden zur Oberflächenmodifikation derartiger Biomaterialien. Ziel dabei ist stets, die vorteilhaften chemischphysikalischen Eigenschaften der Biomoleküle zu erhalten. Deshalb sollte nur die äußerste Oberfläche modifiziert werden, da dadurch deren biologische Wechselwirkungen beeinflußt werden.
Oberflächen-modifizierte Biomaterialien (wie beispielsweise durch Silicon- und Siloxanenthaltende Block-Copolymere, "Silanisierung") finden Verwendung zur Beeinflussung von Zelladhäsion und Wachstum, zur Modifizierung der Blut-Kompatibilität oder zur Kontrolle der Protein-Adsorption (Herabsetzung der Adsorption von Kontaktlinsen, Vorbehandlung von ELISA-Platten). Die Sialinisierung zur Modifizierung von Materialoberflächen kann dabei insbesondere zur Modifizierung hydroxylierter oder Amin-reicher Oberflächen benutzt werden, wie beispielsweise Oberflächen von Glas, Silicium, Germanium, Aluminium, Quarz und vielen Metalloxiden, die reich an Hydroxylgruppen sind. Eine Literatur-Übersicht findet sich in: B.D. Ratner et al. (Hrsg.) Biomaterials Science - An Introduction to Materials in Medicine. Academic Press, San Diego, 1996.
Dabei ergibt sich jedoch das Problem, daß bei den zur Herstellung dieser Modifizierungen angewandten Bedingungen oft schädliche (destruierende) Effekte auf die benutzten Biomaterialien auftreten.
Eine im Vergleich zu den angewandten physikalisch/chemischen Verfahren "milde" Methode stellen die allein auf biochemischen Reaktionen beruhenden Modifikationen von Biomaterialien mit Hilfe des erfindungsgemäßen Verfahrens (Verwendung von rekombinantem/gereinigtem Silicatein) dar (Silicatein-vermittelte enzymatische Synthese von amorphem $SiO_2$-, Siloxan- oder Siloxan-Block-Copolymere-enhaltende Oberflächen).
Die modifizierte Zone auf der Material-Oberfläche sollte in den meisten Fällen so dünn wie möglich sein, da modifizierte Oberflächenschichten, die zu dick sind, die mechanischen und funktionellen Eigenschaften des Materials verändern können. Dies kann durch Variation der Reaktionszeit und der Substrat-Konzentration bei der Silicatein-vermittelten enzymatischen Reaktion auf einfache Weise erreicht werden.
Mittels der Silicatein-vermittelten enzymatischen Reaktion lassen sich eine Vielzahl unterschiedlicher Biomaterialien, die entweder eine Protein (Silicatein)-bindende Oberfläche von Natur aus besitzen bzw. die durch eine vorhergehende Modifikation proteinbindend gemacht wurden, verwenden, und zwar Biomaterialien einschließlich Polymere, Metalle, Keramiken und Gläser.
Silane können zwei Arten von Oberflächen-Strukturen bilden. Sehr dünne (Monolayer) Schichten, falls nur Spuren von Oberflächen-adsorbiertem Wasser vorhanden sind, oder, falls mehr Wasser vorhanden ist, dickere Silan-Schichten, die sowohl aus Si-O Gruppen, die an die Oberfläche gebunden sind, als auch Silan-Einheiten, die ein dreidimensionales, polymeres Netzwerk ausbilden, bestehen. Derartige Modifikationen können beispielsweise durch Behandlung von hydroxylierten Oberflächen mit n-Propyl-trimethoxysilan hergestellt werden.
Allgemeine Literatur: Pleuddemann, E. P. (1980) Chemistry of silane coupling agents. In Silylated Surfaces (D. E. Leyden, Hrsg.) Gordon & Breach, New York, pp.31-53.
Mittels der Silicatein-vermittelten enzymatischen Reaktion lassen sich - unter milden, die Biomaterialien schonenden Bedingungen - ebenfalls verschiedene Arten von Oberflächenstrukturen auf einer Vielzahl unterschiedlicher Biomaterialien bilden, deren Synthese jedoch, da enzymatisch vermittelt, gezielt abläuft.

Insbesondere ergibt sich auch eine Verwendung der rekombinanten oder des aus verschiedenen Quellen gereinigten Silicateins bei der Herstellung von Oberflächen-Modifikationen von Biomaterialien mit Silikon-artigen Eigenschaften (wie Silikon-Brust-Implantate) sowie medizinischer Implantate und Endoprothesen, ebenfalls auch bei Kontaktlinsen.

*5.2. Verwendung von Silicatein zum Einkapseln von Biomolekülen*

Durch die gesteuerte Synthese von $SiO_2$- oder Siloxan-Hüllen mittels des rekombinanten oder des aus verschiedenen Quellen gereinigten Silicateins ergibt sich eine Verwendung zum Einkapseln von Biomolekülen (einschließlich Proteinen und Nucleinsäuren) sowie bioaktiven Molekülen (einschließlich Hormonen, Pharmaka und Cytokinen), mit dem Ziel, deren biologischen Eigenschaften (wie Protease- und Nuclease-Resistenz, Temperatur-Stabilität) oder deren Freisetzung (kontrolliertes "Drug-deliver", ebenfalls auch Ermöglichung eines topischen "Drug-delivery") zu verändern oder zu verbessern.

a) Erhöhung der Proteaseresistenz (und Temperatur-Stabilität) von Proteinen.

Vorgehensweise: Umgebung des Proteins mit einem Silicatein-Mantel (beispielsweise durch Crosslinking), der eine $SiO_2$-Hülle synthetisiert.

b) Erhöhung der Nucleaseresistenz (und Temperatur-Stabilität) von Nucleinsäuren

Vorgehensweise: Umgebung der Nucleinsäure mit einem Silicatein-Mantel (beispielsweise durch Crosslinking), der eine $SiO_2$-Hülle synthetisiert.

Auf die oben geschilderte Weise ist auch die Herstellung von Depot-Formen für Arzneimittel (einschließlich für Peptid/Proteohormone und Cytokine) möglich. Dazu werden die Arzneimittel (einschließlich Peptid/Proteohormone und Cytokine) zunächst modifiziert durch

a) Crosslinking mit Silicatein oder (falls es sich um Proteine handelt)
b) Herstellung von Fusionsproteinen mit Silicatein.

Danach erfolgt die Silicatein-vermittelte Synthese des Kapselmaterials.

WO 9614832 Al 19960523 (US 9514261 19951106) offenbart eine Methode zur Herstellung von synthetischen Liposomen aus Polyphosphaten zur Steigerung der Aufnahme von Arzneimitteln ("Drug-delivery"). Durch Aufbau einer derartigen Liposomen stabilisierenden Hülle mittels Silicatein läßt sich die Effizienz der Methode steigern.

Weiterhin ergibt sich eine Verwendung des rekombinanten oder des aus verschiedenen Quellen gereinigten Silicateins bei der Herstellung neuer Biomaterialien (oder Komposit-Materialien) wie Knochenersatzmaterialien oder Zahnersatzmaterialien durch Co-Synthese von Polysilikaten, Siliconen oder Mischpolymeren (hergestellt mittels des rekombinanten/gereinigten Silicateins) und Polyphosphaten (hergestellt mittels rekombinanter Polyphosphat-Kinase).

*5.3. Verwendung von Silicatein zum Einkapseln von Zellen/Geweben bei Transplantationen*

Die gesteuerte Synthese von $SiO_2$ oder Siloxan-Hüllen mittels des rekombinanten oder des aus verschiedenen Quellen gereinigten Silicateins kann auch zur Einkapselung von Zellen bei Transplantationen verwendet werden (Verbesserung der Biokompatibilität).

Dies kann durch "Coaten" der Zellen mit Silicatein oder durch Expression von Silicatein-Fusionsproteinen auf der Zell-Oberfläche durchgeführt werden.

*5.4. Verwendung von Silicatein zur Oberflächen-Modifikation (Kontaktzonen-Behandlung) von (Silicium)-Halbleitern oder Silicium-Chips und deren Verwendung*

Das rekombinante sowie das aus verschiedenen Quellen gereinigte Silicatein kann auch zur Oberflächen-Modifikation von (Silicium oder Germanium)-Halbleitern oder (Silicium oder Germanium)-Biosensor-Chips verwendet werden. Hierdurch kann eine Verbindung mit Zellen oder anderen aus organischem Material bestehenden Strukturen hergestellt werden. Diese "Matrizes" können zur Messung der elektrischen Eigenschaften von Zellen benutzt werden. Derartige Silicatein-modifizierte Halbleiter (oder Silicium-Mikrochips) können auch als Biosensoren Verwendung finden.

*5.5. Verwendung von Silicatein zur Synthese von Silicium-haltigen Edelsteinen und Halbedelsteinen*

Das rekombinante sowie das aus verschiedenen Quellen gereinigte Silicatein ist in der Lage, amorphes $SiO_2$ zu bilden. Zu den amorphen bzw. feinkristallinen Modifikationen des $SiO_2$ (Opale) zählen Achat, Jaspis, Onyx u.a. Aufgrund der Möglichkeit, mit Hilfe des rekombinanten oder des aus verschiedenen Quellen gereinigten Silicateins unter kontrollierten Bedingungen amorphes $SiO_2$ zu synthetisieren, wobei gezielt Fremdmoleküle/atome eingefügt werden können, ergibt sich folglich die Verwendung des erfindungsgemäßen Verfahrens zur Herstellung der obengenannten und anderer Edelsteine/Halbedelsteine.

Mit Hilfe des rekombinanten oder des aus verschiedenen Quellen gereinigten Silicateins ist es auch möglich, unter kontrollierten Bedingungen dünne SiO- und $SiO_2$-Schichten auf Halbleiter-Unterlagen aufzubringen (zur Herstellung von integrierten Schaltkreisen).

*5.6. Verwendung von Silicatein zur Synthese von Silicium (Gallium-, Zinn- oder Blei-)-Verbindungen (einschließlich sogenannter Sila-Pharmaka)*

Silicium ist bekannt als Spurenelement, das offenbar für die Ausbildung von Bindegewebe und Knochen (Mineralisation) benötigt wird. Die Herstellung von Silicium-organischen Verbindungen als Grundlage für sogenannte Sila-Pharmaka (Pharmaka, bei denen C durch Si ersetzt ist, mit potentiellen Wirkungsänderungen auf den Organismus) ist von medizinischem Interesse [siehe: Chem. unserer Zeit 14, 197-207 (1980), sowie: Bioactive Organo-Silicon Compounds (Topics Curr. Chem. 84), Berlin, Springer 1979)]; die Synthese derartiger Verbindungen unter milden (enzymatischen) Bedingungen ist mittels des erfindungsgemäßen Verfahrens (Verwendung des rekombinanten oder des aus verschiedenen Quellen gereinigten Silicateins) möglich.

Weitere Verwendungen von Silicium-Verbindungen, die mit Hilfe des erfindungsgemäßen Verfahrens unter milden (enzymatischen) Bedingungen synthetisiert werden (Verwendung des rekombinanten oder des aus verschiedenen Quellen gereinigten Silicateins) in Medizin und Kosmetik sind: Verwendung derartig synthetisierter Silicium-Verbindungen als Bestandteil oder Grundlage von Salben, sowie als Bestandteil von Zahnpasten [zur Verwendung in der Kosmetik, siehe: Parfüm. Kosmet. 67, 232-239, 326-336, 384-389(1986); 68,195-203 (1987)].

Silicium-Verbindungen, die mit Hilfe des erfindungsgemäßen Verfahrens unter milden (enzymatischen) Bedingungen synthetisiert wurden (Verwendung des rekombinanten oder des aus verschiedenen Quellen gereinigten Silicateins) lassen sich auch für weitere Zwecke verwenden: Gleitmittel (Kunststoffverarbeitung), Schmiermittel (Kunststoffgetriebe), Schaumdämpfungsmittel, Formtrennmittel, (Hydrophobieren von Glas, Keramik, Textilien, Leder) und Dielektrika (z.B. in Transformatoren).

Die mit Hilfe des erfindungsgemäßen Verfahrens synthetisierten Verbindungen schließen die in der Technik verwendeten Siloxan-Harze wie (mehr oder minder vernetzte) Polymethyl- oder Polymethylphenysiloxane mit ein.

Die durch Kondensation der im Verlauf der Silicatein-vermittelten Katalyse gebildeten Silantriole $RSi(OH)_3$ führen zur Synthese von Blattstruktur-artigen Polymeren der Bruttozusammensetzung $R_2Si_2O_3$. Der Vernetzungsgrad und die Ausdehnung dieser Polymere kann dabei durch kontrollierte Beimischung von Silandiolen und Silanolen variiert werden. Somit lassen sich enzymatisch maßgeschneiderte Siliconstrukturen mit charakteristischen, regulierbaren Eigenschaften erzeugen.

Das Siloxan-Gerüst kann dabei mit verschiedenartigen Kohlenwasserstoff-Resten verknüpft sein. Dadurch lassen sich seine Eigenschaften modifizieren.

Aufgrund ihrer Wärmebeständigkeit und Hydrophobizität können die durch Silicatein-vermittelte Katalyse synthetisierten (erfahrungsgemäß nicht gesundheits-schädlichen) Silicone auch in der kosmetischen Hautpflege und plastischen Chirurgie Verwendung finden.

Allgemeine Literatur: Brinker et al., Polydimethylsiloxane in der Lebens- und Genußmittelindustie, München: Dow Corning 1981.

*5.7. Veränderung der Eigenschaften von Zellen durch Transfektion mit einem Silicatein-Gen/cDNA-enthaltendem Plasmid*

Durch Transfektion von Zellen mit einem Silicatein-Gen/cDNA-enthaltendem Plasmid lassen sich deren Eigenschaften verändern, was eine Verwendung bei der Herstellung von Knochenersatzmaterialien (auch durch Co-"Polymerisation" von Silicatein-synthetisiertem $SiO_2$ und Polyphosphaten, hergestellt durch Transfektion mit einem Polyphosphat-KinasecDNA-enthaltendem Plasmid) ergibt

*5.8. Verwendung von rekombinantem Silicatein zur Synthese von Nano-Strukturen aus amorphem Siliciumdioxid*

Mittels des rekombinantem Silicateins, der rekombinanten Silicatein-Fusionsproteine oder des gereinigtem Silicateins ist es möglich, spezifische zwei- und dreidimensionale, sieb-, netz-, käfig- oder andersartig geformte Strukturen aus amorphem Siliciumdioxid, Siloxanen oder anderen Silicium (IV)- [oder Gallium (IV)-, Zinn (IV)- oder Blei (IV)-] -Verbindungen im Nano-Maßstab zu synthetisieren, wobei mit diesen Enzymen assoziierte Makromoleküle (synthetische Polymere oder Biopolymere) als "Leitschienen" für der Synthese benutzt werden. Derartige Synthesen sind auch unter Verwendung von in Marikultur oder in Aquarien gehaltenen Schwämmen oder anderen Silikatein-produzierenden Organismen sowie mit gentechnologisch veränderten, ursprünglich nicht zur Silikat-Synthese befähigten Organismen möglich. Die gebildeten Strukturen können in der Nanotechnologie angewandt werden (beispielsweise als "Miniatursiebe" für Trennprozesse im Nanomaßstab).

**Erläuterungen zu den Abbildungen:**

Im nachfolgenden sind die erläuternden Legenden zu den beigefügten Zeichnungen aufgeführt. Es zeigt:

**Abbildung 1:**

cDNA mit abgeleiteter Aminosäure-Sequenz für das *Suberites domuncula*-Silicatein.

**Abbildung 2:**

Aminosäure-Sequenz von Silicatein aus dem Schwamm *Suberites domuncula*. Einige Stellen am Protein, die nach Verkürzung der cDNA zu einer starken Erhöhung der Expression des rekombinanten Proteins geführt haben, sind eingezeichnet [—]. Darüber hinaus sind die Aminosäuren der katalytischen Triade (CT) sowie die Cystein-Einheiten, die zu potentiellen Disulfidbrücken führen, markiert (•)

**Abbildung 3:**

Herstellung des rekombinanten Silicatein-Proteins in *E. coli.* Nach dem angegebenen Verfahren wurde aus dem Rohextrakt das reine Silicatein gewonnen. Spur a: Proteinextrakt aus nicht-induzierte Zellen [- IPTG]; Spur b und Spur c: Proteinextrakt aus induzierte Zellen [+ IPTG], 2,5 Stunden (Spur b) und 8 Stunden (Spur c) nach Zugabe von IPTG. Spur d: Löslicher Proteinextrakt des Bakterien-Lysats nach Expression für 8 Stunden. Spur e: Proteinextrakt der "inclusion bodies". Spur f: Gereinigtes Silicatein aus den "inclusion bodies" nach Reinigung des Proteins an einer Ni-NTA Matrix. Das Molekulargewicht des rekombinanten Silicateins ist etwa 33 kDa. Am linken Rand sind die Molekulargewichtsmarker angegeben.

**Abbildung 4:**

(A) Konzentration an Transkripten für Silicatein in Primmorphen von *S. domuncula*. Primmorphe, die nach 5-tägiger Inkubation aus Einzelzellen gebildet worden waren, wurden für 0 (Kontrollen; Kon) bis 5 Tage entweder in Abwesenheit von exogenem Silikat (minus Silikat) oder in Anwesenheit von 60 µM Na-Silikat (plus Silikat) inkubiert. Anschließend wurde die RNA extrahiert und 5 µg der Gesamt-RNA nach ihrer Größe aufgetrennt; nach dem Blot-Transfer wurde die Hybridisierung mit der Silicatein-Sonde von *S. domuncula* (*SUBDOSILICA*) durchgeführt. (B) Effekt von rekombinantem Myotrophin auf die Expression von Silicatein in Primmorphen. Primmorphe wurden für 0 (Kontrollen) bis zu 5 Tagen in Abwesenheit (minus Myotrophin) oder Gegenwart von 1 µg/ml Myotrophin (plus Myotrophin) inkubiert. Dann wurde das Northern-Blotting mit der *SUBDOSILICA*-Sonde zur Bestimmung des Ausmaßes der Expression von Silicatein durchgeführt.

**Abbildung 5:**

Co-Expression von Silicatein und einem Gen, das für eine bioaktive Substanz codiert (Schema). A. Herstellung des Fusionsproteins Silicatein-bioaktives Protein. Die beiden cDNAs [Silicatein und bioaktives Protein] wurden über eine Restriktionsstelle (z. B. *Sal*I) ligiert und anschließend in die Restriktionsstellen *Bam*HI und *Hin*dIII des pQE-30-Vektors eincloniert. Am 5'-Terminus befindet sich das Histidin-Tag. B. Getrennte Protein-Expression. Die beiden cDNAs werden in einem geeigneten Vektor über eine Protease-Spaltstelle eincloniert. Nach Expression und Reinigung wird das Fusionsprotein durch Protease-Verdau getrennt erhalten. C. Getrennte Protein-Expression (als Kassetten-Expression). Die beiden cDNAs werden auf dem gleichen Konstrukt separat exprimiert und über die His-Tags gereinigt.

**Patentansprüche**

1. Verfahren zur Herstellung von rekombinantem Silicatein in einer Wirtszelle, **dadurch gekennzeichnet, daß** es eine Verkürzung des zu exprimierenden rekombinanten Silicateins in der N-terminalen Region seiner codierenden cDNA, die vom Beginn des Propetids bis zur ersten Disulfidbrücke reicht, und/oder die Verkürzung des zu exprimierenden rekombinanten Silicateins durch Einfügung eines Stop-Codons in der von der letzten Disulfidbrücke reichenden C-terminalen kodierenden Region seiner cDNA, und Isolierung des funktionell hochaktiven Enzympräparats umfaßt.

2. Verfahren zur Herstellung von rekombinantem Silicatein in einer Wirtszelle nach Anspruch 1, **dadurch gekennzeichnet, daß** als Wirtszelle Zellen von *Escherichia coli,* Hefen, Säugern oder Primmorphen, wie Meeresschwämme oder Diatomeen verwendet werden.

3. Verfahren zur Herstellung von rekombinantem Silicatein in einer Wirtszelle nach Anspruch 1 oder 2, **dadurch gekennzeichnet, daß** als Wirtszellen Zellen von *Cylindrotheca fusiformis* verwendet werden.

4. Verfahren zur Herstellung von rekombinantem Silicatein in einer Wirtszelle nach einem der vorangehenden Ansprüche, **dadurch gekennzeichnet, daß** das rekombinante Silikatein als (a) Fusionsprotein (chimäres Protein) oder (b) in einer getrennte Protein-Expression (Protease-Spaltstelle) oder (c) in einer zweiten getrennten Protein-Expression (Kassetten-Expression) und in Form einer modifizierten Silicatein-cDNA-Sequenz exprimiert wird.

5. Verfahren zur Herstellung von rekombinantem Silicatein in einer Wirtszelle nach einem der vorangehenden Ansprüche, **dadurch gekennzeichnet, daß** es als weiteren Schritt eine Reinigung des Silicateins mittels polyklonaler Antikörper oder mittels eines Histidin-Tags umfaßt.

6. Verfahren zur Herstellung von rekombinantem Silicatein in einer Wirtszelle nach Anspruch 5, **dadurch gekennzeichnet, daß** der polyklonale Antikörper an eine feste Phase gekoppelt wird.

7. Transgene Wirtszelle, insbesondere Zelle von *Escherichia coli*, Hefen, Säugern oder Primmorphen, wie Meeresschwämme, wie *Suberites domuncula*, oder Diatomeen, wie *Cylindrotheca fusiformis*, wobei die Zelle ein rekombinantes Silicatein exprimiert, das eine Verkürzung des zu exprimierenden rekombinanten Silicateins in der N-terminalen Region seiner codierenden cDNA, die vom Beginn des Propetids bis zur ersten Disulfidbrücke reicht, und/oder die Verkürzung des zu exprimierenden rekombinanten Silicateins durch Einfügung eines Stop-Codons in der von der letzten Disulfidbrücke reichenden C-terminalen kodierenden Region seiner cDNA umfaßt.

## Claims

1. Method for producing recombinant silicatein in a host cell, **characterised in that** said method comprises a truncation of the recombinant silicatein to be expressed in the N-terminal region of its coding cDNA that ranges form the start of the protein to the first disulfide bridge and/or the truncation of the recombinant silicatein to be expressed by introducing a stop codon in the C-terminal coding region of its cDNA starting at the last disulfide bridge, and isolating of the functionally highly active enzyme preparation.

2. Method for producing recombinant silicatein host cell according to claim 1, **characterised in that** cells of *Escherichia coli,* yeasts, mammals or primmorphs, such as marine sponges or diatoms, are used.

3. Method for producing recombinant silicatein in a host cell according to claim 1 or 2, **characterised in that** cells of *Cylindrotheca fusiformis* are used as host cells.

4. Method for producing a recombinant silicatein host cell according to any of the foregoing claims, **characterised in that** the recombinant silicatein is expressed as (a) fusion protein (chimeric protein) or (b) in a separated protein expression (protease cleavage site) or (c) in a second separated protein expression (cassette expression), and in form of a modified silicatein-cDNA-sequence.

5. Method for producing a recombinant silicatein in a host cell according to any of the foregoing claims, **characterised in that** said method comprises, as an additional step, the purification of the silicatein by means of polyclonal antibodies or by means of a histidine tag.

6. Method for producing a recombinant silicatein in a host cell according to claim 5, **characterised in that** the polyclonal antibody is coupled to a solid phase.

7. Transgenic host cell, in particular cells of *Escherichia coli,* yeasts, mammals or primmorphs, such as marine sponges, such as *Suberites domuncula*, or diatoms, such as *Cylindrotheca fusiformis,* wherein said cell expresses a recombinant silicatein that comprises a truncation of the recombinant silicatein to be expressed in the N-terminal region of its coding cDNA that ranges from the start of the propeptide to the first disulfide bridge and/or a truncation of the recombinant silicatein to be expressed by introducing a stop codon in the C-terminal region of the cDNA starting at the last disulfide bridge.

## Revendications

1. Procédé de préparation de silicatéines recombinées dans une cellule hôte, **caractérisé en ce qu'**il comprend un raccourcissement de la silicatéine recombinée à exprimer dans la région N-terminale de son ADNc codant, qui va du début du propeptide jusqu'au premier pont disulfure, et/ou le raccourcissement de la silicatéine recombinée à exprimer par introduction d'un codon d'arrêt dans la région codante C-terminale allant depuis le dernier pont disulfure, de son ADNc, et l'isolement de la préparation enzymatique fonctionnelle très active.

2. Procédé de préparation de silicatéines recombinées dans une cellule hôte selon la revendication 1, **caractérisé en ce que** l'on utilise comme cellule hôte, des cellules de *Escherichia coli*, de levures, de mammifères ou d'organismes primaires, comme des éponges marines ou des diatomées.

3. Procédé de préparation de silicatéines recombinées dans une cellule hôte selon la revendication 1 ou 2, **caractérisé en ce que** l'on utilise comme cellule hôte, des cellules de *Cylindrotheca fusiformis*.

4. Procédé de préparation de silicatéines recombinées dans des cellules hôtes selon l'une quelconque des revendications précédentes, **caractérisé en ce que** la silicatéine recombinée est exprimée comme (a) une protéine de fusion (protéine chimère) ou (b) dans une expression séparée de protéine (site de clivage de protéase) ou (c) dans une deuxième expression séparée de protéine (cassette d'expression) et sous la forme d'une séquence d'ADNc de silicatéine modifiée.

5. Procédé de préparation de silicatéines recombinées dans des cellules hôtes selon l'une quelconque des revendications précédentes, **caractérisé en ce que** comme autre étape, est présente une purification de la silicatéine à l'aide d'un anticorps polyclonal ou à l'aide d'un marqueur histidine.

6. Procédé de préparation de silicatéines recombinées dans des cellules hôtes selon la revendication 5, **caractérisé en ce que** l'anticorps polyclonal est couplé à une phase solide.

7. Cellule hôte transgénique, en particulier cellule de *Escherichia coli*, de levures, de mammifères ou d'organismes primaires, comme des éponges marines, comme *Suberites domuncula*, ou des diatomées, comme *Cylindrotheca fusiformis*, où la cellule exprime une silicatéine recombinée, qui comprend un raccourcissement de la silicatéine recombinée à exprimer dans la région N-terminale de son ADNc codant, qui va du début du propeptide jusqu'au premier pont disulfure, et/ou le raccourcissement de la silicatéine recombinée à exprimer par introduction d'un codon d'arrêt dans la région codante C-terminale allant depuis le dernier pont disulfure, de son ADNc.

# Figur 1

```
GAGGATAGAAAGTCTACAATCTGTAAGGACAATGCTTGTCACAGTGGTAGTACTGGGTCT   60
                                 M  L  V  T  V  V  V  L  G  L   10
ACTGGGGTTTGCTTCTGCAGCCCAGCCCAAGTTTGAATTTGTAGAAGAATGGCAGCTGTG  120
 L  G  F  A  S  A  A  Q  P  K  F  E  F  V  E  E  W  Q  L  W   30
GAAGTCCACTCACTCTAAGATGTACGAGTCACAGTTAATGGAACTCGAAAGACATCTGAC  180
 K  S  T  H  S  K  M  Y  E  S  Q  L  M  E  L  E  R  H  L  T   50
GTGGCTCTCCAATAAGAAATATATCGAGCAACACAATGTCAACTCACACATTTTCGGTTT  240
 W  L  S  N  K  K  Y  I  E  Q  H  N  V  N  S  H  I  F  G  F   70
TACTCTGGCAATGAACCAGTTTGGAGATCTGAGTGAATTGGAGTATGCTAACTATCTTGG  300
 T  L  A  M  N  Q  F  G  D  L  S  E  L  E  Y  A  N  Y  L  G   90
CCAGTATCGCATTGAGGATAAAAAATCTGGCAACTACTCAAAGACTTTTCAGCGTGATCC  360
 Q  Y  R  I  E  D  K  K  S  G  N  Y  S  K  T  F  Q  R  D  P  110
TCTACAGGACTACCCTGAAGCTGTAGACTGGAGAACCAAAGGAGCTGTCACGGCTGTCAA  420
 L  Q  D  Y  P  E  A  V  D  W  R  T  K  G  A  V  T  A  V  K  130
GGACCAGGGAGACTGTGGTGCTAGCTATGCTTTCAGTGCTATGGGTGCTTTGGAGGGTGC  480
 D  Q  G  D  C  G  A  S  Y  A  F  S  A  M  G  A  L  E  G  A  150
TAATGCTTTAGCCAAGGGAAATGCAGTATCTCTCAGTGAACAGAACATCATTGATTGCTC  540
 N  A  L  A  K  G  N  A  V  S  L  S  E  Q  N  I  I  D  C  S  170
GATTCCTTACGGTAACCACGGTTGTCATGGAGGCAATATGTATGATGCTTTTTTGTATGT  600
 I  P  Y  G  N  H  G  C  H  G  G  N  M  Y  D  A  F  L  Y  V  190
CATCGCTAACGAGGGGGTCGATCAGGACAGTGCATATCCATTTGTAGGAAAGCAATCCAG  660
 I  A  N  E  G  V  D  Q  D  S  A  Y  P  F  V  G  K  Q  S  S  210
CTGCAACTATAATAGTAAATACAAAGGTACATCAATGTCGGGGATGGTGTCAATCAAAAG  720
 C  N  Y  N  S  K  Y  K  G  T  S  M  S  G  M  V  S  I  K  S  230
TGGTAGTGAGTCTGACTTACAAGCAGCTGTTTCAAACGTTGGCCCTGTATCTGTTGCTAT  780
 G  S  E  S  D  L  Q  A  A  V  S  N  V  G  P  V  S  V  A  I  250
TGATGGTGCTAACAGTGCCTTCAGGTTTTACTACAGTGGTGTCTATGACTCATCACGATG  840
 D  G  A  N  S  A  F  R  F  Y  Y  S  G  V  Y  D  S  S  R  C  270
CTCTAGTAGTAGTCTTAACCACGCAATGGTAGTCACTGGATACGGATCATACAATGGGAA  900
 S  S  S  S  L  N  H  A  M  V  V  T  G  Y  G  S  Y  N  G  K  290
AAAATACTGGCTGGCCAAGAATAGCTGGGGAACTAACTGGGGTAACAGTGGCTATGTGAT  960
 K  Y  W  L  A  K  N  S  W  G  T  N  W  G  N  S  G  Y  V  M  310
GATGGCTCGCAACAAGTACAACCAGTGTGGAATTGCTACCGATGCATCTTATCCCACCCT 1020
 M  A  R  N  K  Y  N  Q  C  G  I  A  T  D  A  S  Y  P  T  L  330
ATAAACTTATATATATATAGTCTTAGAAACATTATCCTTTTCTTTACCCTTGTCTCTATA 1080
GGCCATAGAGTGATTGTAGGCTGTTTGCATTTGATGACTGTATATACCCTATCATTTTTT 1140
GTGATTCTATCTGATTAAAAATCCCATACCCGACCAAACCATCAATTTATCAAATCATGA 1200
```

# Figur 2

SILCA_SUBD : MLVTVVVLGLLGFASAAQPKFEFVEEWQLWKSTHSKMYESQIMELERHLTWLSNKK : 56

Propeptid

SILCA_SUBD : YIEQHNVNSHIFGFTLAMNQFGDLSELEYANYLGQYRIEDKKSGNYSKTFQRDPLQ : 112

CT

reifes Protein

SILCA_SUBD : DYPEAVDWRTKGAVTAVKDQGDCGASYAFSAMGALEGANALAKGNAVSLSEQNIID : 168

SILCA_SUBD : CSIPYGNHGCHGGNMYDAFLYVIANEGVDQDSAYPFVGKQSSCNYNSKYKGTSMSG : 224

CT

SILCA_SUBD : MVSIKSGSESDLQAAVSNVGPVSVAIDGANSAFRFYYSGVYDSSRCSSSSLNHAMV : 280

CT

SILCA_SUBD : VTGYGSYNGKKYWLAKNSWGTNWGNSGYVMMARNKYNQCGIATDASYPTL : 330

**Figur 3**

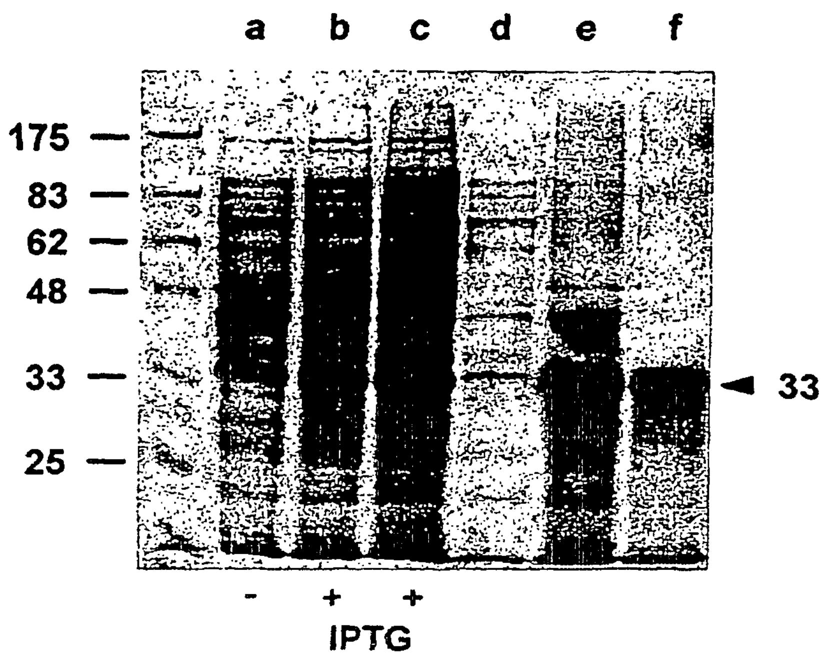

**Figur 4**

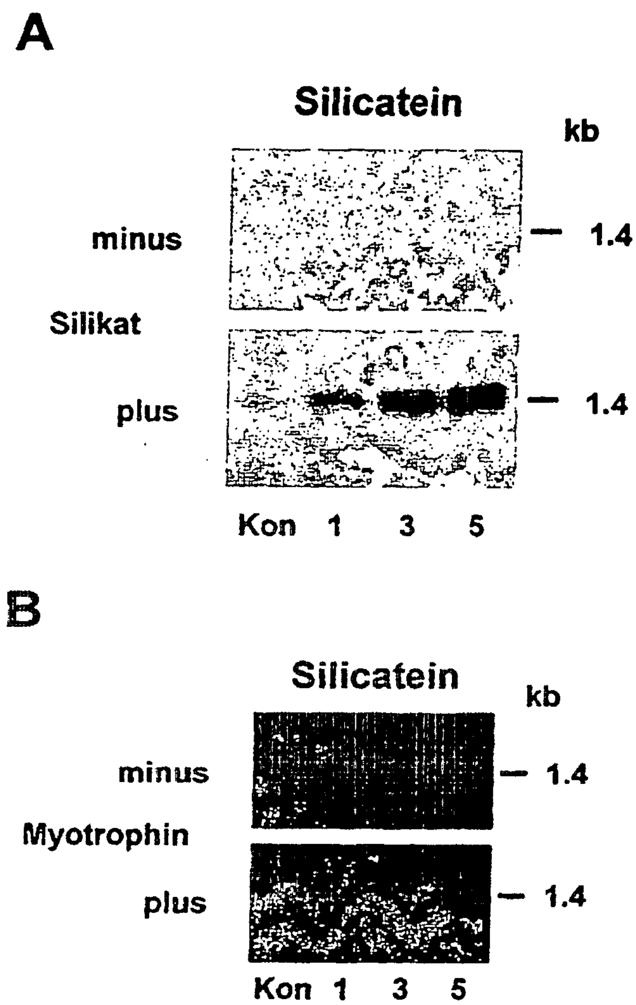
A
Silicatein
kb
minus
Silikat
plus
1.4
1.4
Kon 1 3 5
B
Silicatein
kb
minus
Myotrophin
plus
1.4
1.4
Kon 1 3 5

# Figur 5

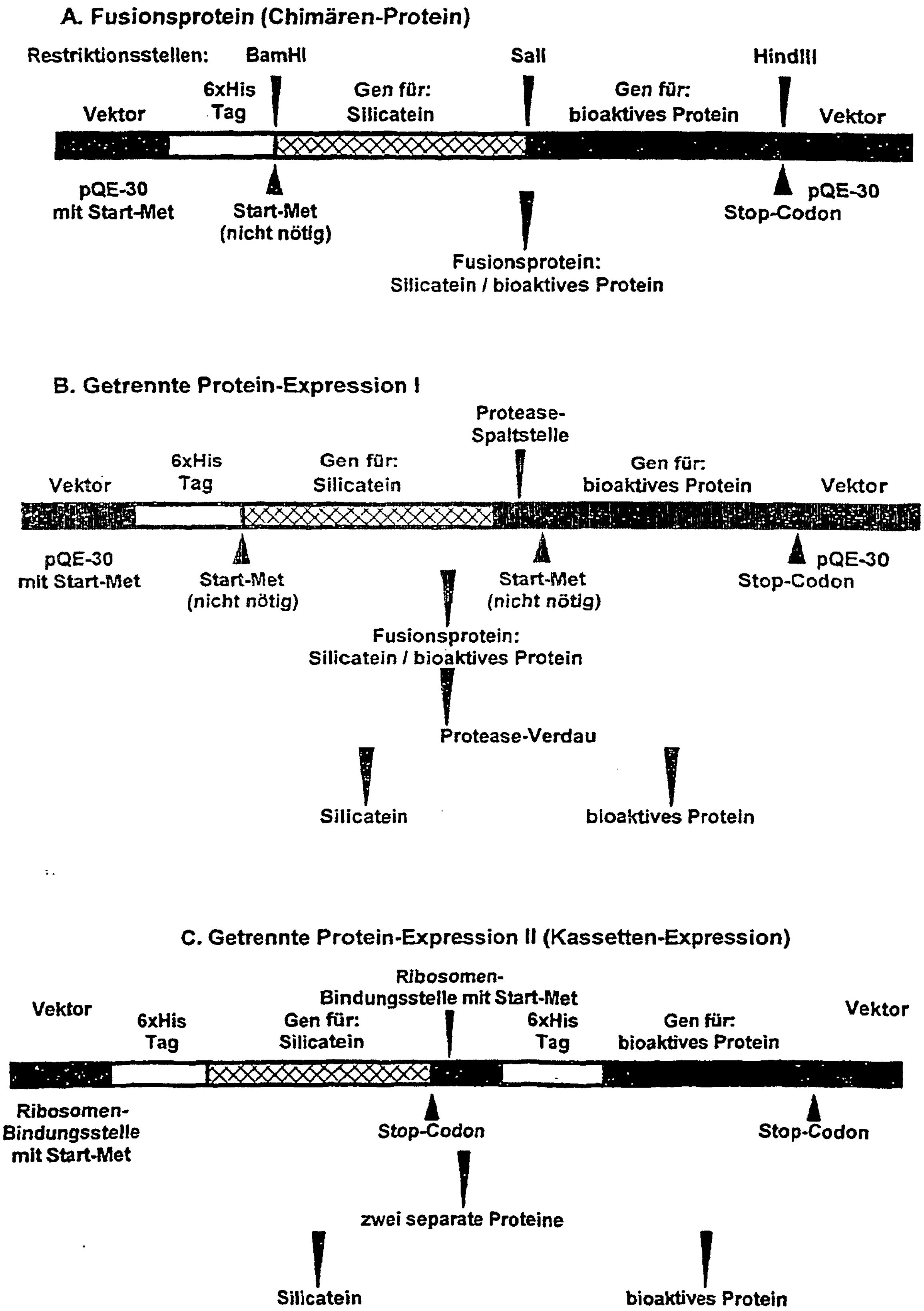
A. Fusionsprotein (Chimären-Protein)
Restriktionsstellen: BamHI
SalI
HindIII
Vektor
6xHis Tag
Gen für: Silicatein
Gen für: bioaktives Protein
Vektor
pQE-30 mit Start-Met
Start-Met (nicht nötig)
pQE-30 Stop-Codon
Fusionsprotein: Silicatein / bioaktives Protein
B. Getrennte Protein-Expression I
Protease-Spaltstelle
Vektor
6xHis Tag
Gen für: Silicatein
Gen für: bioaktives Protein
Vektor
pQE-30 mit Start-Met
Start-Met (nicht nötig)
Start-Met (nicht nötig)
pQE-30 Stop-Codon
Fusionsprotein: Silicatein / bioaktives Protein
Protease-Verdau
Silicatein
bioaktives Protein
C. Getrennte Protein-Expression II (Kassetten-Expression)
Ribosomen-Bindungsstelle mit Start-Met
Vektor
6xHis Tag
Gen für: Silicatein
6xHis Tag
Gen für: bioaktives Protein
Vektor
Ribosomen-Bindungsstelle mit Start-Met
Stop-Codon
Stop-Codon
zwei separate Proteine
Silicatein
bioaktives Protein

SEQUENCE LISTING

<110> Müller, Werner E.G.
<120> Silicatein-vermittelte Synthese von amorphen Silikaten und Siloxanen und ihre Verwendung

<130> M30116PCT
<140> PCT/EP01/08423
<141> 2001-07-20
<160> 2

<170> PatentIn version 3.1

<210> 1
<211> 1200
<212> DNA
<213> Suberites domuncula

<400> 1

```
gaggatagaa agtctacaat ctgtaaggac aatgcttgtc acagtggtag tactgggtct     60
actggggttt gcttctgcag cccagcccaa gtttgaattt gtagaagaat ggcagctgtg    120
gaagtccact cactctaaga tgtacgagtc acagttaatg gaactcgaaa gacatctgac    180
gtggctctcc aataagaaat atatcgagca acacaatgtc aactcacaca ttttcggttt    240
tactctggca atgaaccagt ttggagatct gagtgaattg gagtatgcta actatcttgg    300
ccagtatcgc attgaggata aaaaatctgg caactactca aagacttttc agcgtgatcc    360
tctacaggac taccctgaag ctgtagactg gagaaccaaa ggagctgtca cggctgtcaa    420
ggaccaggga gactgtggtg ctagctatgc tttcagtgct atgggtgctt tggagggtgc    480
taatgcttta gccaagggaa atgcagtatc tctcagtgaa cagaacatca ttgattgctc    540
gattccttac ggtaaccacg gttgtcatgg aggcaatatg tatgatgctt ttttgtatgt    600
catcgctaac gagggggtcg atcaggacag tgcatatcca tttgtaggaa agcaatccag    660
ctgcaactat aatagtaaat acaaaggtac atcaatgtcg gggatggtgt caatcaaaag    720
tggtagtgag tctgacttac aagcagctgt ttcaaacgtt ggccctgtat ctgttgctat    780
tgatggtgct aacagtgcct tcaggtttta ctacagtggt gtctatgact catcacgatg    840
ctctagtagt agtcttaacc acgcaatggt agtcactgga tacggatcat acaatgggaa    900
aaaatactgg ctggccaaga atagctgggg aactaactgg ggtaacagtg gctatgtgat    960
gatggctcgc aacaagtaca accagtgtgg aattgctacc gatgcatctt atcccaccct   1020
ataaacttat atatatatag tcttagaaac attatccttt tctttaccct tgtctctata   1080
ggccatagag tgattgtagg ctgtttgcat ttgatgactg tatataccct atcatttttt   1140
gtgattctat ctgattaaaa atcccatacc cgaccaaacc atcaatttat caaatcatga   1200
```

<210> 2
<211> 330
<212> PRT
<213> Suberites domuncula

<400> 2

```
Met Leu Val Thr Val Val Val Leu Gly Leu Leu Gly Phe Ala Ser Ala
1               5                   10                  15

Ala Gln Pro Lys Phe Glu Phe Val Glu Glu Trp Gln Leu Trp Lys Ser
            20                  25                  30

Thr His Ser Lys Met Tyr Glu Ser Gln Leu Met Glu Leu Glu Arg His
        35                  40                  45

Leu Thr Trp Leu Ser Asn Lys Lys Tyr Ile Glu Gln His Asn Val Asn
    50                  55                  60

Ser His Ile Phe Gly Phe Thr Leu Ala Met Asn Gln Phe Gly Asp Leu
65                  70                  75                  80

Ser Glu Leu Glu Tyr Ala Asn Tyr Leu Gly Gln Tyr Arg Ile Glu Asp
                85                  90                  95

Lys Lys Ser Gly Asn Tyr Ser Lys Thr Phe Gln Arg Asp Pro Leu Gln
            100                 105                 110

Asp Tyr Pro Glu Ala Val Asp Trp Arg Thr Lys Gly Ala Val Thr Ala
        115                 120                 125

Val Lys Asp Gln Gly Asp Cys Gly Ala Ser Tyr Ala Phe Ser Ala Met
    130                 135                 140

Gly Ala Leu Glu Gly Ala Asn Ala Leu Ala Lys Gly Asn Ala Val Ser
145                 150                 155                 160

Leu Ser Glu Gln Asn Ile Ile Asp Cys Ser Ile Pro Tyr Gly Asn His
                165                 170                 175

Gly Cys His Gly Gly Asn Met Tyr Asp Ala Phe Leu Tyr Val Ile Ala
            180                 185                 190

Asn Glu Gly Val Asp Gln Asp Ser Ala Tyr Pro Phe Val Gly Lys Gln
        195                 200                 205

Ser Ser Cys Asn Tyr Asn Ser Lys Tyr Lys Gly Thr Ser Met Ser Gly
    210                 215                 220

Met Val Ser Ile Lys Ser Gly Ser Glu Ser Asp Leu Gln Ala Ala Val
225                 230                 235                 240
```

```
Ser Asn Val Gly Pro Val Ser Val Ala Ile Asp Gly Ala Asn Ser Ala
                245                 250                 255

Phe Arg Phe Tyr Tyr Ser Gly Val Tyr Asp Ser Ser Arg Cys Ser Ser
            260                 265                 270

Ser Ser Leu Asn His Ala Met Val Val Thr Gly Tyr Gly Ser Tyr Asn
        275                 280                 285

Gly Lys Lys Tyr Trp Leu Ala Lys Asn Ser Trp Gly Thr Asn Trp Gly
    290                 295                 300

Asn Ser Gly Tyr Val Met Met Ala Arg Asn Lys Tyr Asn Gln Cys Gly
305                 310                 315                 320

Ile Ala Thr Asp Ala Ser Tyr Pro Thr Leu
                325                 330
```